# EUROPÄISCHE PATENTSCHRIFT

(11) **EP 2 556 155 B1**
(45) Veröffentlichungstag und Bekanntmachung des Hinweises auf die Patenterteilung: **19.08.2015**
(21) Anmeldenummer: 11713269.6
(22) Anmeldetag: 07.04.2011
(51) Int. Cl.: C12N 15/10

(54) **VERFAHREN UND KIT ZUR TRENNUNG VIRALER UND PROKARYONTISCHER VON EUKARYONTISCHEN NUKLEINSÄUREN**
METHOD AND KIT FOR SEPARATING VIRAL AND PROKARYOTIC NUCLEIC ACIDS FROM EUKARYOTIC NUCLEIC ACIDS
PROCÉDÉ ET KIT POUR SÉPARER DES ACIDES NUCLÉIQUES VIRAUX ET PROCARYOTES D'ACIDES NUCLÉIQUES EUCARYOTES

(30) Priorität: 08.04.2010 AT 5672010
(43) Veröffentlichungstag der Anmeldung: 13.02.2013
(73) Patentinhaber: Greiner Bio-One GmbH, 72636 Frickenhausen (DE)
(72) Erfinder: RUDORFER, Walter, 4271 St. Oswald/Freistadt (AT)
(74) Vertreter: Burger, Hannes
(86) Internationale Anmeldenummer: PCT/EP2011/055449
(87) Internationale Veröffentlichungsnummer: WO 2011/124653

(56) Entgegenhaltungen:
- EP-A1- 1 524 320
- WO-A1-2005/085440
- US-A1- 2008 187 916
- US-A1- 2009 137 024

## Beschreibung

Die Erfindung betrifft ein Verfahren zur Trennung viraler und prokaryontischer Nukleinsäuren von eukaryontischen Nukleinsäuren aus einer biologischen Probe, insbesondere Zellsuspension, umfassend die Schritte a) Zentrifugieren und Resuspendieren der eukaryontischen Zellen, b) Lyse der Zellen, c) Neutralisation der Zelllysats und d) Abtrennen der ausgefällten eukaryontischen Nukleinsäuren sowie die Verwendung eines Kits umfassend einen Resuspensions-, Lyse- und Neutralisationspuffer zur Trennung viraler und prokaryontischer Nukleinsäuren von eukaryontischen Nukleinsäuren aus einer biologischen Probe, insbesondere Zellsuspension.

Dem raschen Nachweis von Bakterien und Viren in eukaryontischen Zellen, unabhängig ob in in-vitro oder in-vivo, kommt immer eine größere Bedeutung zu. Infektionen in-vivo können für einen Menschen oder ein Tier lebensbedrohlich sein bzw. Krankheiten hervorrufen und in-vitro vor allem in der Biopharmaindustrie die Produktion von Impfstoffen bzw. allgemein von Biopharmazeutika gefährden. Eine rasche Ursachenabklärung ist Voraussetzung für eine entsprechende Behandlung bzw. Einstellung der Produktion. Um aber bereits geringe Mengen einer Kontamination nachweisen zu können, bedarf es sehr sensitiver und auch spezifischer Methoden. Da bereits geringste Mengen der Infektion bzw. Kontamination nachgewiesen werden sollen, ist Material in welchem sich die Infektion bzw. Kontamination befindet in großem Überschuss vorhanden.

Die Kontamination von Zellkulturen beispielsweise durch Mollicutes ist ein weit verbreitetes und ernst zu nehmendes Problem in der biologischen Forschung und biopharmazeutischen Industrie. Es gibt viele negative Effekte von Mollicutes-, insbesondere Mycoplasmenkontaminationen, auf Zellkulturen, welche beispielsweise die Proteinsynthese, den Zellstoffwechsel oder die Zellmorphologie verändern. Mycoplasmen sind als parasitär lebende Bakterien die Ursache für zahlreiche Krankheiten beim Menschen, Tieren und Pflanzen. In der Regel töten Bakterien aus der Klasse der Mollicutes ihren Wirt jedoch nicht ab. Vielmehr verursachen sie chronische Infektionen, was für eine gute Anpassung an die Wirte spricht, und verkörpern damit eine sehr erfolgreiche Art des Parasitismus. Neben den pathogenen Eigenschaften der Mycoplasmen spielt die Infektion von Zellkulturen mit Mycoplasmen eine wichtige Rolle. Der Nachweis von Mycoplasmen kann über verschiedene Methoden erfolgen.

Mit dem Produkt CytoInspect® der Anmelderin konnte bisher rasch die Mollicutesspezies und die Quelle der Mollicuteskontamination in Zellkulturüberständen und -medien aber nicht aus Zellsuspensionen bestimmt werden.

Aus dem Stand der Technik ist das Verfahren "alkalische Lyse" zur Isolation von Plasmid-DNA (zirkuläre DNA) aus Bakterien bekannt (Birnboim and J. Doly. A rapid alkaline extraction procedure for screening recombinant plasmid DNA. Nucleic Acids Research. Vol. 7(6); 1979 und Maniatis, T., E. F. Fritsch, and J. Sambrook. 1982. Molecular cloning: a laboratory manual, p. 90-91. Cold Spring Harbor Laboratory, Cold Spring Harbor, N.Y.). Die alkalische Lyse ist die Standardmethode zur Isolation von Plasmid-DNA aus prokaryontischer DNA, wobei die prokaryontische DNA ausgefällt wird und die Plasmid-DNA in Lösung bleibt. In "Molecular Cloning" wird auch beschrieben, dass bei harschen Lysemethoden (Detergentien + Kochen oder alkalischer Lyse) bakterielle DNA denaturiert wird. Während der Lyse werden bakterielle Proteine, Zellwandbruchteile und denaturierte chromosomale DNA in große Komplexe mit dem Detergens umhüllt. Diese Komplexe können effizient durch Austausch der Natriumionen mit Kaliumionen ausgefällt werden. Nachdem das denaturierte Material beispielsweise durch Zentrifugation entfernt wird, befindet sich die Plasmid DNA im Überstand.

Ferner beschreiben zwei Publikationen (Nascimento R. Elmiro, D. A. (1999). Plasmid in Mycoplasma species from goats and sheeps and their preliminary typing. Revista de Microbiologia und Andrew D. Bergemann, Jane C. Whitley, and Lloyd R. Finch, Homology of Mycoplasma Plasmid pADB201 and Staphylococcal Plasmid pE194, J. of Bacteriology, Jan. 1989, P. 593-595 171, No. 1) die Verwendung der alkalischen Lyse um in Mycoplasmen natürlich vorkommende Plasmid DNA von genomischer Mycoplasmen DNA zu trennen, dabei wird die prokaryontische DNA abgeschieden und nur die Plasmid DNA bleibt in Lösung, wodurch die prokaryontische (chromosomale) DNA für die weitere Analyse nicht mehr verfügbar ist.

Auch die WO 2005/085440 A1 beschreibt ein Verfahren, wo die Trennung und/oder Anreicherung prokaryonter DNA aus Untersuchungsproben mit hohem Anteil eukaryonter DNA, insbesondere von Patienten mit Infektionen, ermöglicht wird. Erfindungsgemäß wird dies durch ein Protein erreicht, das nicht methylierte CpG-Motive bindet, wobei es eine 25%-ige bis 35%-ige Homologie, insbesondere etwa 27,6 %-ige Homologie, zum Wildtyp-CPGB-Protein aufweist und gegenüber diesem maximal bis zur Länge der Bindungsstelle verkürzt ist.

Die US 2009/137024 A1 beschreibt ein Verfahren zur Trennung von viraler, bakterieller und fungaler DNA von eukaryontischer DNA durch Ausnutzung der Unterschiede in Bezug auf das CpG Motiv. Das CpG Motiv kommt im prokaryontischen Genom 100-mal häufiger vor als im eukaryontischen Genom. Das CpG Motiv ist an eine feste Matrix gebunden. Nachdem die Zellen lysiert werden, schmilzt der DNA-Doppelstrang auf und die einzelsträngige DNA bindet an das immobilisierte CpG-Motiv. Anschließend wird gewaschen und nochmals erhitzt und die zuvor gebundene einzelsträngige DNA wieder vom CpG-Motiv gelöst und gesammelt.

Die US 2008/187916 A1 beschreibt spezifische Primer und ein Amplifikationsverfahren zum Nachweis von mit Mycoplasmen infizierten Zellen. Ferner werden die unterschiedlichen Schmelztemperaturen der PCR-Produkte genutzt um eine Identifikation und Zuordnung zu ermöglichen.

Die EP 1 524 320 A1 beschreibt ein Verfahren zum Nachweis von Mikroorganismen aus eukaryontischen Zellen mittels eines Mikroarrays, wobei die Nukleinsäuren mit aus dem Stand der Technik bekannten Methoden präpariert werden. Es wird mRNA isoliert, in cDNA revers transkribiert und diese mittels PCR oder in-vitro Transkription amplifiziert.

Aufgabe der vorliegenden Erfindung ist es ein Verfahren zur Verfügung zu stellen, womit aus einem großen Volumen eukaryontischer Zellsuspension auch geringe Mengen viraler und prokaryontischer Nukleinsäuren isoliert werden können, insbesondere um eine Nachweisgrenze von <10 cfu/ml (colony forming units/ml) der Bakterien oder Viren zu erreichen.

Die Aufgabe der Erfindung wird jeweils eigenständig durch ein Verfahren zur Trennung viraler und/oder prokaryontischer Nukleinsäuren von eukaryontischen Nukleinsäuren aus einer biologischen Probe, insbesondere eukaryontischen Zellsuspension, umfassend die Schritte in der Reihenfolge a) Resuspendieren der Zellen einer Zellsuspension bzw. der Zellen nach Zentrifugation der Zellen in Gegenwart eines Chelat-Bildners, insbesondere EDTA oder EDTA in Kombination eines Saccharids, b) Lyse der Zellen durch chemische Lyse, wie alkalische Lyse, enzymatische Lyse und/oder Kochlyse und/oder mechanische Lyse, wobei die Lyse in Gegenwart eines Detergens, insbesondere SDS, und einer Base durchgeführt wird, c) Neutralisation des Zelllysats in Gegenwart zumindest eines Salzes bzw. Stoffes, das/der die Löslichkeit von Proteinen reduziert, insbesondere eines kosmotropen Salzes, insbesondere Kalium- oder Natriumacetat bzw. Ammoniumsulfat, wo die Proteinfällung erfolgt, und d) Abtrennen der ausgefällten eukaryontischen Nukleinsäuren sowie Gewinnung der viralen und/oder prokaryontischen Nukleinsäuren, die in Lösung bleiben; sowie die Verwendung eines Kits umfassend einen Resuspensionspuffer mit Chelatbildner und gegebenenfalls ein Saccharid und RNAse, Lysepuffer umfassend zumindest eine Base und ein Detergens und Neutralisationspuffer zur Trennung viraler und/oder prokaryontischer Nukleinsäuren von eukaryontischen Nukleinsäuren aus einer biologischen Probe, insbesondere eukaryontischen Zellsuspension, gelöst. Vorteilhaft dabei erweist sich, dass die eukaryontische DNA, Proteine und Zellmembranbestandteile abgeschieden werden, während die viralen und prokaryontischen Nukleinsäuren in Lösung bleiben. Da das Übermaß an eukaryontischer DNA abgetrennt wird, kann in weiterer Folge eine Nukleinsäureamplifikationstechnik (Nucleic acid amplification technique - NAAT), wie der Polymerase-Kettenreaktion (PCR) der gewonnenen Nukleinsäuren, durchgeführt werden. Zur erfolgreichen Anwendung von nucleic acid amplification techniques (NAAT), wie z.B. PCR, wird möglichst reine DNA bzw. cDNA, etc. als Vorlage benötigt. Verunreinigungen wie Proteine, Fette oder andere organische Substanzen oder auch ein Übermaß an Fremd-DNA (i.e. nicht zu amplifizierende DNA) beeinträchtigen eine erfolgreiche PCR bis zum Totalausfall. Man spricht auch von PCR-Inhibitoren. Versuche im eigenen Labor zeigten, dass Fremd-DNA in einer Konzentration von ca. >3 µg /PCR die Reaktion unterbinden. Je kleiner das Verhältnis zwischen Ziel-DNA und Fremd-DNA umso stärker ist der inhibitorische Einfluss der Fremd-DNA.

Ungünstige Ziel- versus Fremd-DNA-Verhältnisse sind in der PCR-Diagnostik häufig anzutreffen. Beispiele dafür sind: PCR aus Blut, Geweben aller Art, Bodenproben, forensische PCR, Nachweise oder Hygienekontrollen in der Lebensmittel- oder Pharmaindustrie, etc.

Als Beispiel sei folgendes angeführt: Eine Anforderung der Pharmaproduktion ist Mycoplasmenkontaminationen im Ausmaß von <10 colony forming units (cfu)/ml aus mindestens 10 ml Zellsuspensionen feststellen zu können. Die Zellzahl der eukaryontischen Zellen in 10 ml einer Fermenterkultur beträgt ca. 10^8 Zellen. Dies entspricht einer DNA-Menge von ca. 580 µg, also einer Menge bei der keine PCR mehr stattfindet. 10 cfu/ml Mycoplasmenzellen in 10^8 eukaryontischen Zellen entspricht einem Nukleinsäureverhältnis von 1:3x10^10. Nimmt man als Berechnungsgrundlage ein PCR-Amplifikat von 300 Nukleotiden Länge so berechnet sich sogar ein Verhältnis von 1:10^14. Bei solchen Verhältnissen zwischen Ziel- und Fremd-DNA ist eine PCR-Amplifikation unmöglich und damit auch die Anwendung der PCR für Hygienekontrollen aus Zellsuspensionen.

Vorteilhaft beim erfindungsgemäßen Verfahren und Kit erweist sich zudem, dass die Isolation der viralen und prokaryontischen Nukleinsäuren aus eukaryontischen Zellsuspensionen einfach und schnell möglich ist und eine Inhibition weder durch zelluläre Proteine noch durch Zellmembranbestandteile noch durch eukaryontische DNA nachfolgender Amplifikation stattfindet. Zudem ist von Vorteil, dass auch intrazelluläre virale und prokaryontische Nukleinsäuren nachgewiesen werden können.

Die Resuspension der Zellen kann direkt in der Zellsuspension erfolgen bzw. wird bevorzugt vor der Resuspension eine Zentrifugation durchgeführt, um die Zellen zu konzentrieren/pelletieren und anschließend zur resuspendieren. Die Resuspension wird in Gegenwart eines Chelatbildners, insbesondere EDTA, und gegebenenfalls eines Saccharids durchgeführt, wobei durch den Chelatbildner die Zellmembran destabilisiert wird und DNAsen inhibiert werden. Das Saccharid stabilisiert die Osmolarität.

Die Lyse kann durch chemische, wie alkalische Lyse, enzymatische Lyse, und/oder Kochlyse und/oder mechanische Lyse erfolgen, wodurch neben intranukleärer Nukleinsäuren auch zytoplasmatische Nukleinsäuren isoliert werden können.

Vorzugsweise wird die Lyse in Gegenwart eines Detergens, insbesondere SDS, und einer Base durchgeführt, wobei das Detergens die Zellmembran durchlöchert und die Base die Zellwand lockert und Nukleinsäuren freisetzt und denaturiert. Zelluläre DNA wird linearisiert, wohingegen zirkuläre DNA unverändert bleibt.

Die Neutralisation wird in Gegenwart zumindest eines Salzes bzw. Stoffes, das/der die Löslichkeit von Proteinen reduziert, insbesondere eines kosmotropen Salzes, insbesondere Kalium- oder Natriumacetat bzw. Ammoniumsulfat, durchgeführt, wodurch zirkuläre DNA wieder renaturieren kann, wohingegen zelluläre DNA als einzelsträngige Nukleinsäure denaturiert bleibt und präzipitiert wird. In einer alternativen Ausführungsform kann die Neutralisation auch in Gegenwart eines chaotropen Salzes, insbesondere Guanidiniumsalzes, wie Guanidiniumhydrochlorid, erfolgen. Die eukaryontischen Zellbestandteile, Proteine sowie eukaryontischen genomischen Nukleinsäuren fallen aus und können z.B. mittels Zentrifugation abgetrennt werden.

Die viralen und prokaryontischen Nukleinsäuren verbleiben in Lösung und werden nach der Neutralisation gereinigt, insbesondere präzipitiert (z.B. Alkoholfällung oder Säulchenreinigung - NucleoSpin® Plasmid), sodass die Nukleinsäuren im Zellpellet vorliegen oder gereinigt von der Säule eluiert werden können und den weiteren Arbeitsschritten, wie einer Amplifikation, insbesondere PCR, zugeführt werden können.

Von Vorteil ist, dass durch eine Amplifikation der prokaryontischen Nukleinsäuren aus einer Zellsuspension mit einer Zelldichte von 10^5 bis 10^10 Zellen/ml eine Nachweisgrenze <10 cfu/ml bzw. <10 Kopien/ml erreicht werden kann, wodurch eine sehr sensitive Methode zum Nachweis von Kontaminationen von Viren und Prokaryonten in eukaryontischen Zellen, insbesondere menschlichen und tierischen Zellen bzw. Zellkulturen, zur Verfügung steht. Vorteilhaft ist auch, dass die viralen und prokaryontischen Nukleinsäuren aus bis zu 100 ml Zellsuspension nachgewiesen werden können. Das Verhältnis der Anzahl der prokaryontischen bzw. viralen Ziel-DNA zur Anzahl der störenden eukaryontischen DNA Moleküle weist immer ein Verhältnis <1, insbesondere um mehrere Größenordnungen, auf.

In einer möglichen Ausführungsvariante der Erfindung kann Mollicutes DNA von eukaryontischer DNA durch das Verfahren "alkalische Lyse" separiert werden, wodurch auf vorteilhafte Weise zelluläre Komponenten und die DNA der eukaryontischen Zellen soweit abgetrennt werden, dass ein negativer Effekt weder für die nachfolgende Extraktion noch Amplifikation auftritt.

Von Vorteil erweist sich auch, dass in Verbindung mit NAAT der Nachweis von Mollicutes, insbesondere Mycoplasmen, in einer Konzentration von weniger als 5 cfu/ml aus einer Zellsuspension einer Zelldichte von bis zu 10^10 eukaryontischen Zellen/ml möglich ist bzw. 5 cfu/ml Mycoplasmen aus 100 ml einer Zellsuspension mit 10^9 Zellen bzw. 10 ml mit 10^8 Zellen möglich ist.

Die Vorteile für die Verwendung des erfindungsgemäßen Kits entsprechen den zuvor angeführten Vorteilen des erfindungsgemäßen Verfahrens.

Im Zuge der Erfindung wird unter biologischer Probe zelluläres Material verstanden, aus dem Nukleinsäuren isoliert werden können, insbesondere Zellen aus Zellkultur, Zelllinien, Zellen aus Blut, Harn, Speichel oder anderen Körperflüssigkeiten, sowie Zellen aus Geweben, z.B. Biopsien, Abstrichen, etc.

Erfindungsgemäß werden Nukleinsäuren, insbesondere virale bzw. prokaryontische DNA eines Organismus von der DNA eines eukaryontischen Organismus abgetrennt. Virale bzw. prokaryontische intra- oder extrazelluläre DNA eines Organismus ist verpackt, dh es sind Strukturen bzw. Umhüllungen vorhanden.

Ferner wird unter "alkalischer Lyse" das eingangs erwähnte Verfahren zur Isolation von intrazellulär vorhandener Plasmid-DNA aus Bakterien verstanden. Plasmid-DNA ist nackt, dh sie weist keinen Proteinmantel auf. Erfindungsgemäß wird dieses Verfahren mit Adaptierungen zur Trennung von viraler und/oder prokaryontischer Nukleinsäuren von eukaryontischen Nukleinsäuren aus eukaryontischen Zellsuspensionen verwendet. Überraschenderweise konnte gezeigt werden, dass eine Abtrennung eukaryontischer Nukleinsäuren von prokaryontischen bzw. viralen Nukleinsäuren, die in Lösung bleiben, möglich ist, wohingegen in der Literatur beschrieben wird, dass mit dem Verfahren "alkalische Lyse" die prokaryontische DNA abgeschieden wird und nur die Plasmid-DNA in Lösung bleibt.

In diversen Versuchen, wo einige nachfolgend kurz beschrieben werden, wurde gezeigt, dass der Nachweis von Mycoplasmen DNA aus Suspensionskulturen erst ab einer Nachweisgrenze von 500 cfu/ml möglich ist.

Mit dem DNA Trennungssystem des Looxster® Universal Kits kann prokaryontische DNA aus einem DNA-Gemisch isoliert werden. Dieser Kit ermöglicht die Trennung von prokaryontischer DNA von einem Gemisch mit eukaryontischer DNA. Dabei wird eine Affinitätschromatographie durchgeführt, bei der nicht methylierte DNA-Motive der bakteriellen DNA von einem Protein gebunden und somit isoliert werden. Durch die hohe DNA-Abtrennung, welche durch photometrische Messwerte bestätigt sind (es bleiben ca. 7% des ursprünglichen DNA-Gehalts nach der Looxster®-Behandlung der Probe zurück), kommt es zu DNA Verlusten. Durch den hohen DNA Verlust während der LOOXSTER^{®} Anwendung ist es erklärbar, warum drei Arten (Mycoplama gallisepticum, Mycoplasma fermentans und Mycoplasma hyorhinis) erst bei 500 cfu/ml detektierbar sind.

In einer weiteren Extraktionsmethode zur Isolierung von Mycoplasmen DNA aus Suspensionskulturen wurde NP 40 (Tergitol-Typ NP-40 (Nonyl phenoxylpolyethoxylethanol)) getestet. NP-40 soll die Zytoplasmamembran von Mycoplasmen und eukaryontischen Zellen aufbrechen. Die Kernmembran der eukaryontischen Zellen soll intakt bleiben. Mycoplasmen DNA ist im Zytoplasma und eukaryontische DNA im Zellkern lokalisiert. Durch NP-40 soll die Mycoplasmen DNA freigesetzt werden und die eukaryontische DNA im Zellkern bleiben und somit eine Trennung der DNAs ermöglichen. In der Praxis zeigte sich allerdings der gegenteilige Effekt. Die Anzahl der Mycoplasmen im Zellpellet und im Zellüberstand ist annähernd gleich. Würde NP-40 tatsächlich funktionieren, müsste die Konzentration der Mycoplasmen im Überstand deutlich höher sein. Somit ist eine Trennung von Mycoplasmen DNA und eukaryontischer DNA mit NP-40 nicht möglich.

Auch eine Trennung von Mollicutes DNA von menschlichen und humanen Zellkulturen mittels Filter wurde getestet, wobei die Mycoplasmen durch die Filter nicht aufgehalten werden, jedoch relativ große eukaryontische Zellen schon. Große Mengen von Zellen verstopfen allerdings die Membran.

Die Trennung von Mycoplasmen von eukaryontischen Zellen durch Zentrifugation wurde ebenfalls evaluiert (eukaryontische Zellen sollen schwerer sein als Mycoplasmen). Nachteilig dabei erweist sich, dass intrazelluläre Mycoplasmen nicht detektiert werden und somit die Sensitivität gering ist.

Im Unterschied zum Stand der Technik, wo Plasmide von prokaryontischer DNA getrennt werden, werden mit der erfindungsgemäßen Methode virale und prokaryontische Nukleinsäuren von eukaryontischer DNA getrennt. So ist neben dem Unterschied Prokaryonten/Eukaryonten auch das Größenverhältnis der zu trennenden DNAs (beispielsweise Mollicutes, insbesondere Mycoplasmen, DNA von eukaryontischer DNA) beim erfindungsgemäßen Verfahren deutlich anders wie jenes aus dem Stand der Technik bekannte Verhältnis. Zwar sind Mollicuteschromosomen, insbesondere Mycoplasmenchromosomen, ca. einen Faktor 3-5 kleiner als die von E. coli, aber dieser Größenunterschied ist nicht so groß, dass sich im Vergleich zu E. coli (aus dem die meisten Plasmide gewonnen werden) Mycoplasmenchromosomen wie Plasmide verhalten würden. Plasmide sind meist um einen Faktor 50-100 kleiner als E. coli Genome. Und für zwei Mollicutesspezii Acholeplasma laidlawii und Spiroplasma citri liegt das Verhältnis der Genomgröße zu jener von E. coli sogar nur bei einem Faktor von 2-3.

Nachfolgend sind Genomgrößen einzelner Bakterienspezies beispielhaft angeführt.

| Spezies | Genomgröße [kb] |
|---|---|
| Acholeplasma laidlawii | 1680 |
| Spiroplasma citri | 1820 |
| Streptococcus mutans NN2025 | 2013 |
| Staphylococcus epidermidis ATCC 12228 | 2499 |
| Staphylococcus haemolyticus JCSC1435 | 2685 |
| Enterococcus faecalis OG1RF | 2739 |
| Clostridium difficile CD 196 | 4110 |
| Bacillus subtilis | 4214 |
| Escherichia Coli | 4557 |
| Mycoplasma spp. | 580 - 1358 |

Das erfindungsgemäße Verfahren und der Kit können beispielsweise zur Trennung von bakterieller, insbesondere prokaryontischer DNA der Bakterienarten Staphylococcus haemolyticus, Clostridium difficile, Streptococcus mutans, Staphylococcus epidermidis, Lactobacillus casei, Enterococcus faecalis, Staphylococcus saprophyticus, Streptococcus mitis, Eubacterium nodatum, Bacillus subtilis, Streptococcus gordonii, Enterococcus faecium, E. coli, Peptostreptococcus micros, Streptococcus agalactiae, Streptococcus oralis, Streptococcus pneumoniae, Streptococcus pyogenes, Veillonella parvula, von eukaryontischer DNA verwendet werden. Selbstverständlich ist auch die Trennung der DNA anderer Bakterienarten von eukaryontischer DNA möglich.

Das erfindungsgemäße Verfahren und der Kit können auch für die Trennung prokaryontischer DNA von eukaryontischer DNA aus Human- oder anderen eukaryontischen Zellen verwendet werden. So kann beispielsweise die bakterielle DNA von Clostridium perfringens, Enterococcus faecalis, Enterococcus faecium, Listeria monocytogenes, Propionibacterium acnes, Staphylococcus aureus, Staphylococcus epidermidis, Streptococcus agalactiae, Streptococcus dysgalactiae, Subspecies equisimilis, Streptococcus pneumoniae, Streptococcus pyogenes, Coagulase negative Staphylococcus, Acinetobacter baumannii, Enterobacter aerogenes, Enterobacter cloacae, Escherichia coli, Haemophilus influenzae, Kingella kingae, Klebsiella oxytoca, Klebsiella pneumoniae, Neisseria meningitidis, Proteus mirabilis, Proteus vulgaris, Pseudomonas aeruginosa, Salmonella enterica subspecies enterica, Serratia marcescens, Stenotrophomonas maltophilia, Bacteroides fragilis,Campylobacter jejuni, Campylobacter coli, Enterobacteriaceae, Neisseria sp. non-meningitidis, Bacteroides fragilis, Bacteroides vulgatus, Bacteroides thetaiotaomicron; Coagulase negative Staphylococcus: S. haemolyticus, S. hominis S. lugdunensis, S. saprophyticus, S. warneri, S. xylosus; Enterobacteriaceae: Citrobacter amalonaticus, Citrobacter braakii, Citrobacter freundii, Citrobacter koseri, Enterobacter hormaechei, Enterobacter sakazakii, Kluyvera intermedia, Morganella morganii, Pantoea agglomerans, Providencia rettgeri, Providencia stuartii, Yersinia enterocolitica, Yersinia pseudotuberculosis; Neisseria sp. non-meningitidis: N. gonorrhoeae, N. subflava, N. sicca, N. cinerea, N. elongata subspecies nitroreducens, N. flavescens, N. lactamica, N. zoodegmatis; Salmonella enterica subspecies enterica serovars: Enteritidis, Oranienburg, Othmarschen, Panama, Paratyphi, Stanley, Typhi, Typhimurium, Virchow group A,B,C,D von humaner bzw. anderer eukaryontischer genomischer DNA getrennt werden.

Exemplarisch für das erfindungsgemäße Verfahren und den Kit sei auch die Trennung viraler DNA, wie beispielsweise der Retroviren, Herpesviren, Flaviviren, Papovaviren, Paramyxoviren, Parvoviren, Picornaviren, Caliciviren, Rhabdoviren, Reoviren, Togaviren insbesondere Viren wie Influenza, Parainfluenza, Sindbis, Bovine viral diarrhea, Murine leukemia, Westnil, Human immunodeficiency, Human Papilloma, Bovine herpes, Bovine Viral Diarrhea, Pseudo rabies, Reo 3, Encephalomyocarditis, Hepatitis A, Hepatitis B, Hepatitis C, Polio type 1, Simian 40 (SV-40), Minute virus of mice, Vesicular Stomatitis, Porcine parvo, Avian Leukosis, Epstein-Barrvirus, Herpes simplex, Encephalitis, Cytomegalo, Vesivirus 2117, etc. von eukaryontischer DNA angeführt.

Mögliche Beispiele für eukaryontische DNA sind neben humaner und tierischer genomischer DNA auch Zelllinien, die in der Biotechnologie oder Biopharmaindustrie eingesetzt werden. Beispielhaft hierfür sind Zelllinien, wie AS-30D, MDCK, MCF-7, MFM-223, HCC1937, KPL-1, T-47D, UM-UC-3,TCC-SUP, J82, T-24, Vm-Cub 1, HL-60, PL21, THP-1, Molt-4, NALM-1, WSU-NHL, DOHH-2, SU-DHL-4, U-251 MG, U373 MG, U-87 MG, SNB-19, DAOY, D425, D341,UW 228-2, HEP-G2, 1184, A-431, CaCo-2, HaCaT, HEK 293, HEK 293 EBNA , HeLa, Huvec-c, HDMEC, MG-63, RPMI-2650, SaOs-2, HP-6017, GT2, BHK-21, C2C12, CHO-K1, CHO-XM, COS-1, CS-1, Lcells, MC3T3-E1, MDCK, mocha, NIH-3T3, PC12, PK-1, REF52, etc. Selbstverständlich ist auch die Trennung der DNA anderer Zelllinien von viraler und prokaryontischer Nukleinsäure möglich.

### Ausführungsbeispiel 1:

Nachfolgend ist ein Ausführungsbeispiel des erfindungsgemäßen Verfahrens angeführt. Mollicutes DNA, im speziellen Acholeplasma (A.) axanthum, A. laidlawii, A. modicum, A. morum, A. oculi, A. vituli, Mycoplasma (M.) alkalescens, M. arginini, M. arthritidis, M. bovigenitalium, M. bovirhinis, M. bovis, M. bovoculi, M. buccale, M. californicum, M. canadense, M. canis, M. eqhirhinis, M. faucium, M. fermentans, M. flocculare, M. gallinaceum, M. gallinarum, M. gallisepticum, M. genitalium, M. glycophilium, M. hominis, M. hyopharyngis, M. hyopneumoniae, M. hyorhinis, M. hyosynoviae, M. orale, M. pirum, M. pneumoniae, M. pulmonis, M. salivarium, M. synoviae, S. citri, S. kunkelii, U. diversum/urealyticum, aus einer AS-30D Zellsuspension mit einer Zelldichte von 10^7 Zellen/ml wird extrahiert, wobei eine Nachweisgrenze von 5 cfu/ml erreicht wird.

Zehn ml Zellsuspension werden 20 Minuten bei 4500 g bei 20°C zentrifugiert. Der Überstand wird verworfen und das Zellpellet durch Vortexen resuspendiert. Anschließend werden 500 µl Resuspensionspuffer (50 mM Glucose, 25 mM Tris/HCl pH 8,0, 10 mM EDTA) inklusive 20 µl interne PCR Kontrolle (künstliches Plasmid, das Primerbindungsstellen zu den PCR-Primern hat, sonst aber (abgesehen von der Capture-Sonde) eine völlig beliebige Sequenz haben kann) beigemengt und gevortext. Eine Menge von 20 µl Proteinase K (23 mg/ml) wird beigegeben und abermals gevortext.

Im nächsten Schritt werden 500 µl Lysepuffer (10 ml 0,2 N NaOH, 10 % SDS) zugegeben und vorsichtig geschwenkt, 10 Minuten bei 56°C inkubiert und anschließend 1 Minute bei 4500 g bei 20°C zentrifugiert. Hierbei wird der Effekt genutzt, dass Prokaryonten (Mycoplasmen) zirkuläre DNA und Eukaryonten lineare DNA aufweisen.

Anschließend werden 600 µl Neutralisationspuffer (3 M Na Acetat pH 4,8 bis 5,2) zugegeben und vorsichtig geschwenkt und 1 Minute bei 4500 g zentrifugiert. Der Überstand wird in eine neues Röhrchen überführt und abermals 10 Minuten bei mindestens 11.000g zentrifugiert.

Nachfolgend kann die DNA über Säulchen, wie beispielsweise Silikatsäulchen, aus dem Überstand isoliert und vom Säulchen eluiert werden bzw. über eine Isopropanolfällung gewonnen werden. Durch die Verwendung der vorab angeführten Methode und Volumina der Puffer werden die zellulären Komponenten soweit wie möglich bereits abgetrennt, wodurch auch auf vorteilhafte Weise die nachfolgende Extraktion der DNA erleichtert wird, weil beispielsweise ein Verklumpen der Säulchen verhindert werden kann.

Anschließend wird eine PCR mit spezifischen Primern für Mollicutes durchgeführt, wie sie aus dem Stand der Technik bekannt ist (z.B. aus dem CytoInspect®-Kit der Anmelderin, Cat.-No. 464 060), die Amplifikate auf einem Microarray hybridisiert, anschließend detektiert und das Ergebnis evaluiert.

Nachfolgend ist in tabellarischer Form das Ergebnis des Mollicutesnachweis mittels im Ausführungsbeispiel 1 beschriebenen Verfahrens zur Extraktion der DNA und anschließender PCR-Amplifikation der Eluate dargestellt. Die SNR-Daten (signal to noise ratio) fünf wichtiger Mollicutesstämme sind dargestellt.

| | Mycoplasma orale | | Acholeplasma laidlawii | | Mycoplasma pneumoniae | | Mycoplasma fermentano | | Mycoplasma hyorhinis | | Spiroplasma citrii/kunkelii | |
|---|---|---|---|---|---|---|---|---|---|---|---|---|
| | 1 | 2 | 1 | 2 | 1 | 2 | 1 | 2 | 1 | 2 | 1 | 2 |
| AS-30D | 993 | 899 | 725 | 713 | 1601 | 1282 | 116 | 142 | 214 | 120 | 320 | 108 |
| RPMI | 1208 | 653 | 931 | 723 | 1424 | 1617 | 432 | 441 | 240 | 190 | 618 | 128 |

Zwei verschiedene Medien, einerseits RPMI-Zellkulturmedium ohne zelluläre Komponenten, wie Proteine und Zellfragmente, und andererseits 10 ml der AS-30D Zellsuspension mit einer Zellmenge von 10^8 Zellen sind verglichen. Bei 5 cfu/ml können deutliche Signale detektiert werden. Gezeigt sind je 2 Ergebnisse aus unabhängigen Experimenten (1 bzw. 2). Das Ergebnis zeigt, dass die PCR auch bei großen Volumina und komplexen Proben, welche hohe Mengen eukaryontischer DNA und Proteine enthalten, sensitiv ist. Die AS-30D Zelllinie ist repräsentativ auch für andere Zelllinien.

Mit dem oben beschriebenen Ausführungsbeispiel kann eine Nachweisgrenze von 5 cfu/ml erreicht werden. Von der Europäischen Pharmacopoeia ist ein Minimum von 10 cfu/ml gefordert.

### Ausführungsbeispiel 2:

Im nachfolgenden Ausführungsbeispiel werden Staphylococcus aureus, Staphylococcus epidermidis, Coagulase negative Staphylococcus: S. haemolyticus, S. hominis S. lugdunensis, S. saprophyticus, S. warneri, S. xylosus aus der Zellkulturlinie MDCK mit einer Zellsuspension mit 10^8 Zellen/ml nachgewiesen. Es wird eine Nachweisgrenze von 5 cfu/ml erreicht.

Zwanzig ml Zellsuspension werden 15 Minuten bei 4000 g bei 22°C zentrifugiert. Der Überstand wird verworfen und das Zellpellet durch Vortexen resuspendiert. Anschließend werden 400 µl Resuspensionspuffer (1 M Tris HCL pH 8,0, 0,5 M EDTA pH 8,0, 10mg/ml RNAseA) inklusive 10 µl interne PCR Kontrolle beigemengt und gevortext.

Im nächsten Schritt werden 500 µl Lysepuffer (2 M KOH, 20 % CHAPS) zugegeben und vorsichtig geschwenkt, 10 Minuten bei 56°C inkubiert und anschließend 1 Minute bei 4500 g bei 20°C zentrifugiert.

Anschließend werden 600 µl Neutralisationspuffer (5M Kaliumacetat, Eisessig, Endresultat ist eine 3 M Kalium- und 5M Acetatkonzentration) zugegeben und vorsichtig geschwenkt und 1 Minute bei 4500 g zentrifugiert. Der Überstand wird in eine neues Röhrchen überführt und abermals 10 Minuten bei mindestens 11.000g zentrifugiert.

Anschließend wird die DNA wie im ersten Ausführungsbeispiel beschrieben eluiert, mit für Staphylococcusinfektionen spezifischen Primern amplifiziert (John Welsh and Michael McClelland, Nucleic Acids Research, Vol. 18, No. 24, 7213, 1990), die Amplifikate auf einem Microarray hybridisiert und das Ergebnis detektiert und evaluiert.

### Ausführungsbeispiel 3:

Im nachfolgenden Ausführungsbeispiel wird Neisseria meningitidis aus humanen Mukosazellen mit einer Zelldichte von 10^6 Zellen/ml nachgewiesen. Es wird eine Nachweisgrenze von 5 cfu/ml erreicht.

Hundert ml Zellsuspension werden 15 Minuten bei 4000 g bei 22°C zentrifugiert. Der Überstand wird verworfen und das Zellpellet durch Vortexen resuspendiert. Anschließend werden 500 µl Resuspensionspuffer (50 mM Glucose, 25 mM Tris/HCl pH 8,0, 10 mM EDTA, 10 mg/ml RNAseA) inklusive 20 µl interne PCR Kontrolle beigemengt und gevortext.

Im nächsten Schritt werden 500 µl Lysepuffer (2 M NaOH, 20 % SDS) zugegeben und vorsichtig geschwenkt, 10 Minuten bei 56°C inkubiert und anschließend 1 Minute bei 4000 g bei 20°C zentrifugiert.

Anschließend werden 600 µl Neutralisationspuffer (3 M Na Acetat pH 4,8 bis 5,2) zugegeben und vorsichtig geschwenkt und 1 Minute bei 4000 g zentrifugiert. Der Überstand wird in eine neues Röhrchen überführt und abermals 10 Minuten bei mindestens 10.000g zentrifugiert.

Anschließend wird die DNA wie im ersten Ausführungsbeispiel beschrieben eluiert, mit spezifischen Primern für Neisseria meningitidis amplifiziert (Greisen K. et al.; Journal of Clinical Microbiology, Feb. 1994, p. 335-351), die Amplifikate auf einem Microarray hybridisiert und das Ergebnis detektiert und evaluiert.

### Ausführungsbeispiel 4:

Es wird Human Papillomavirus aus der humanen Zelllinie LC5 mit einer Zelldichte von 10^9 Zellen/ml nachgewiesen. Es wird eine Nachweisegrenze von 5 Kopien/ml erreicht.

Fünfzig ml Zellsuspension werden 20 Minuten bei 4500 g bei 20°C zentrifugiert. Der Überstand wird verworfen und das Zellpellet durch Vortexen resuspendiert. Anschließend werden 500 µl Resuspensionspuffer (100 mM Tris/HCl pH 8,0, 10 mM DMSA) inklusive 20 µl interne PCR Kontrolle beigemengt und gevortext.

Im nächsten Schritt werden 500 µl Lysepuffer (1M LiOH, 1 % Triton-X 100) zugegeben und vorsichtig geschwenkt, 10 Minuten bei 56°C inkubiert und anschließend 1 Minute bei 4500 g bei 20°C zentrifugiert.

Anschließend werden 600 µl Neutralisationspuffer (4 M K-Phosphat) zugegeben und vorsichtig geschwenkt und 1 Minute bei 4000 g zentrifugiert. Der Überstand wird in eine neues Röhrchen überführt und abermals 10 Minuten bei mindestens 15.000g zentrifugiert.

Anschließend wird die DNA mittels Alkoholfällung präzipitiert, mit spezifischen Primern wie in der EP 2 062 983 A2 offenbart, hybridisiert und das Ergebnis detektiert und evaluiert.

### Ausführungsbeispiel 5:

Es wird Vesivirus aus der humanen Zelllinie HEK mit einer Zelldichte von 10^9 Zellen/ml nachgewiesen. Es wird eine Nachweisegrenze von 10 Kopien/ml erreicht.

Hundert ml Zellsuspension werden 20 Minuten bei 4500 g bei 20°C zentrifugiert. Der Überstand wird verworfen und das Zellpellet durch Vortexen resuspendiert. Anschließend werden 500 µl Resuspensionspuffer (100 mM Tris/HCl pH 8,0, 10 mM DMPS) inklusive 20 µl interne PCR Kontrolle beigemengt und gevortext.

Im nächsten Schritt werden die Zellen durch Kochlyse aufgebrochen (J. Sambrook and D.W. Russel. 2001. Molecular cloning: a laboratory manual, Preparation of DNA by Boiling Lysis (1.43 und A1.16), Third Edition, Cold Spring Harbor Laboratory, Cold Spring Harbor, N.Y) und anschließend 1 Minute bei 4500 g bei 20°C zentrifugiert.

Anschließend werden 600 µl Neutralisationspuffer (4 M K-Phosphat) zugegeben und vorsichtig geschwenkt und 1 Minute bei 4000 g zentrifugiert. Der Überstand wird in eine neues Röhrchen überführt und abermals 10 Minuten bei mindestens 15.000g zentrifugiert.

Anschließend wird die DNA mittels Cäsiumchlorid-Zentrifugation gewonnen, mit spezifischen Primern für Vesivirus amplifiziert (ViralSEQ™ Vesivirus Assay), das Amplifikat auf einem Microarray hybridisiert und das Ergebnis detektiert und evaluiert.

Einige mögliche Zusammensetzungen für die erfindungsgemäßen Puffer sind im nachfolgenden exemplarisch aufgelistet:

### Resuspensionspuffer:

50 mM Glucose, 25 mM Tris/HCl pH 8,0, 10 mM EDTA oder
1 M Tris HCL pH 8,0, 0,5 M EDTA pH 8,0, 10mg/ml RNAseA oder
50 mM Glucose, 25 mM Tris/HCl pH 8,0, 10 mM EDTA, 10 mg/ml RNAseA

### Lysepuffer:

10 ml 0,2 N NaOH, 10 % SDS oder
2 M NaOH, 20 % SDS oder
0,2 N NaOH, 1 % SDS

### Neutralisationspuffer:

3 M Na Acetat pH 4,8 bis 5,2 oder
5M Kaliumacetat, Eisessig, Endresultat ist eine 3 M Kalium- und 5M Acetatkonzentration

Anstelle des in den Ausführungsbeispielen 1 bis 4 konkret beschriebenen Verfahrens kann die Trennung der bakteriellen bzw. viralen DNA bzw. RNA von eukaryontischen Nukleinsäuren auch mittels nachfolgendem Verfahren erfolgen, wobei eine Nachweisgrenze von 10 cfu/ml bzw. 10 Kopien/ml erzielt wird. 1. Bakterielle Kultur Pelletieren, 2. Resuspendierung in eiskalter Alkaline lysis solution I (oder Lysozym siehe Birnboim and Doly 1979), 3. Zugabe von Alkaline lysis solution II, auf Eis lagern, 4. Zugabe von Alkaline lysis solution III, auf Eis lagern, 5. Zentrifugieren bei 4°C, Überstand gewinnen, 6. optional Phenol-Chloroform Extraktion, 7. Gewinnung der viralen bzw. bakteriellen DNA umfassend sie Schritte a. Fällung der DNA bzw. RNA mit Ethanol oder Isopropanol, b. Zentrifugieren, c. Entfernen des Überstands, d. Wiederholung der Schritte a-c, e. Entfernen des Rest-Ethanols durch Lufttrocknen, f. Aufnehmen der Plasmid DNA in TE-Buffer (pH8,0) und RNAse A. Die Alkaline lysis solution I besteht aus 50 mM Glucose, 25 nm Tris-Cl (pH 8.0), 10 nM EDTA (pH 8.0), die Alkaline lysis solution II besteht aus 0,2 N NaOH, 1% (w/v) SDS und die Alkaline lysis solution III besteht aus 5M Kaliumazetat, Eisessig (Endresultat ist eine 3 M Kalium- und eine 5 M Azetat Konzentration).

Die nachfolgende Methode ist eine Alternative zu der vorab beschriebenen Methode und somit zu dem in den Ausführungsbeispielen 1 bis 4 beschriebenen Verfahren. Sie ist sanfter, beschädigt daher die bakterielle bzw. virale DNA bzw. RNA weniger, hat aber auch eine geringere Ausbeute, wodurch auch die Nachweisgrenze sinkt. Zuerst wird die bakterielle Kultur pelletiert und in einer Tris-Sucrose Lösung resuspendiert. In den nachfolgenden Schritten erfolgt die Zugabe von Lysozym mit 0,25 M EDTA (pH 8,0), Zugabe von 10 % SDS, Zugabe von NaCl (Endkonzentration 1 M) und die Gewinnung der bakteriellen bzw. viralen DNA bzw. RNA umfassend die Schritte: a. Entfernen der hoch-molekularen DNA und bakteriellen "Debris" durch Zentrifugation, Gewinnen des Überstands, b. Ix Phenol-Chloroform Extraktion und 1x Chloroform Extraktion, c. Ethanolfällung der wässrigen Phase, d. Zentrifugation und Waschen (70 % Ethanol), Trocknen, g. Aufnehmen der Plasmid DNA in TE-Puffer (pH8,0).

In alternativen Ausführungsformen sind aber auch andere Pufferzusammensetzungen bzw. für die Lyse auch andere Methoden wie oben stehend angeführt möglich.

So kann zur Resuspension anstelle des EDTA auch ein anderer Chelatbildner, wie z.B. DMSA, DMPS, etc., verwendet werden, wobei allerdings nicht alle Chelatbildner mit allen Basen kompatibel sind, da beinahe alle zweiwertigen Kationen mit EDTA unlösliche Komplexe bilden, und somit die EDTA Menge sofort von der Base aufgebraucht wäre. Die Auswahl des Chelatbildners kann die Lyse verbessern.

Für die Lyse kann neben der alkalischen Lyse können auch andere Lysemethoden wie Kochlyse, enzymatische Lyse und mechanische Lyse verwendet werden. Die relevanten Parameter im Falle der alkalischen Lyse sind die Base, wodurch ein bestimmter pH-Wert erreicht wird, und die Wahl und Konzentration des Detergens. Neben NaOH als Base kann auch beispielsweise KOH verwendet werden. Aber auch andere Basen wie LiOH, RbOH, CsOH, Ca(OH)₂, Sr(OH)₂, Ba(OH)₂ können verwendet werden. Es können aber auch andere organische Basen, wie beispielsweise stickstoffhaltige Basen, zur Zelllyse verwendet werden.

Das verwendete Detergens hängt unter anderem stark vom zu lysierenden Zelltyp ab. Als Detergens kann sowohl ein ionischen Detergens, wie SDS oder Deoxycholinsäure, als auch ein zwitterionisches Detergens, wie CHAPS (3-[(3-Cholamidopropyl)dimethylammonio]-2-hydroxy-1-propanesulfonate) oder ein nichtionisches Detergens, wie Triton-X, Nonidet P40 (NP-40), n-Octylglucoside als auch Ethyltrimethylammoniumbromid oder Cetyltrimethylammoniumbromid (CTAB) verwendet werden. Mögliche Konzentrationen im Lysepuffer des ionischen Detergens liegen zwischen 0,01 und 0,5 % bzw. des nichtionischen Detergens zwischen 0,1 und 2%.

Eine bedeutende Rolle kommt vielleicht auch dem Kationentausch von Natrium zu Kalium des Dodecylsulfates zu (im Falle, dass im Neutralisationspuffer Kaliumionen enthalten sind), das wesentlich schlechter löslich ist. Die Löslichkeit von Kalium Dodecyl-Sulfat (KDS) ist wesentlich geringer als die von Natrium Dodecyl-Sulfat (SDS= sodium Dodecyl-Sulfat). Durch die Zugabe von Kalium-Azetat fällt das wesentlich weniger lösliche KDS aus.

Für die Neutralisation, wo die Proteinfällung erfolgt, wäre eine Theorie, dass die Trennung der eukaryontischen DNA von der viralen und prokaryontischen Nukleinsäure, insbesondere DNA, hauptsächlich auf diesem Schritt beruht, da eukaryontische DNA mit den DNAgebundenen Proteinen mit ausfällt, während die prokaryontische DNA an die keine/nur wenige Protein gebunden sind, nicht ausfällt.

Im wesentlichen gäbe es drei Parameter, die die Trennung der eukaryontischen DNA von der prokaryontischen DNA bewirken würden: 1. pH-Wert, 2. kosmotrope Wirkung, die die Proteinfällung steuert und 3. das Detergens.

Die beiden ersten Parameter werden durch die Auswahl des Salzes für den Neutralisationspuffer gesteuert. Die Säurekonstante des schwachen Anions definiert den pH-Wert und die kosmotrope Eigenschaft des Salzes (Kation + Anion) zusammen mit dem Detergens bestimmen die Proteinlöslichkeit bzw. -ausfällung. Der pH-Wert könnte auch die Löslichkeit der Nukleinsäuren beeinflussen. Die Löslichkeit von Proteinen ist am isoelektrischen Punkt (IP) am geringsten, daher muss der pH-Wert für die Proteinfällung so eingestellt werden, dass er möglichst nahe am IP liegt. Die Wirkung der kosmotropen Salze ist meist verstärkt, wenn der pH-Wert niedriger ist als der IP.

Es gibt einige Standardmethoden (Aussalzen) für die Proteinausfällung/reinigung, wieTrichloressigsäure (TCA)-Fällung oder Ammonimum-Sulfat-Fällung, wobei durch sequentielle Änderung, z.B. der Ammonium-Konzentration, auch eine Fraktionierung erreicht werden kann. Die Auswahl der Salze kann auch gemäß der Hofmeister Serie erfolgen, die sich von links nach rechts von kosmotrop nach chaotrop entwickelt. Kosmotrope Salze stabilisieren Proteine und hydrophobe Aggregate in Lösung, wohingegen sie die Löslichkeit von hydrophoben Aggregaten reduzieren. Sie machen Proteine damit weniger löslich (ähnlich Ammonium-Sulfat Protokoll).

Die kosmotrope Wirkung (=Aussalzen, Verringerung der Löslichkeit) wird durch Herabsetzen der Temperatur noch verstärkt.

Je geringer die Proteinkonzentration in der Lösung ist, desto höher ist die notwendige Salzkonzentration um ein Aussalzen zu bewirken. (Dies gilt aber nicht für alle Proteine). Bevorzugt wird im neutralen bzw. leicht sauren Bereich mit starken Konzentrationen von NaCl (5.3 M), Na₂SO₄ (1.9 M), (NH₄)₂SO₄ (4M) bei zum Teil unterschiedlichen Temperaturen (0-25°C) gearbeitet. MgSO₄, Na- oder K-Phosphat (3-3.7 M), Na-Acetat können auch oft als erster Fällungsschritt zur Vorreinigung eingesetzt werden.

Weitere für die Zelllyse wichtige Reagentien liegen in folgenden Konzentrationen vor: die Salzkonzentration liegt meist zwischen 0 und 1M und die Konzentration divalenter Kationen zwischen 0 und 10mM und die EDTA Konzentrationen zwischen 0 und 5mM. Der pH-Wert beträgt meist zwischen 4,0 und 14,0.

Die DNA Reinigung, wie Alkoholfällung (Ethanol, Isopropanol, etc.), Fällung mit Polyethylenglycol, Cäsiumchlorid-Zentrifugation oder Säulchenreinigung (Säulenchromatographie), am Ende des erfindungsgemäßen Verfahrens können wichtig für die Entfernung von Inhibitoren sein, sind aber wahrscheinlich nicht wesentlich für die Trennung viraler und prokaryontischer Nukleinsäuren, insbesondere DNA, von eukaryontischer DNA, sondern diese erfolgt bereits vorher.

Um kleine Nukleinsäurefragmente zu entfernen kann eine Zentrifugation mit NaCl durchgeführt werden (J. Sambrook and D.W. Russel. 2001. Molecular cloning: a laboratory manual, Third Edition, Cold Spring Harbor Laboratory, Cold Spring Harbor, N.Y. 1.78). Plasmid-DNA-Präparationen sind oft mit kleinen Nukleinsäure-Fragmenten (RNA und DNA) verunreinigt, welche von bakteriellen Chromosomen oder gebrochenen Plasmiden stammen können. Obwohl das Gewicht klein ist, kann die Anzahl sehr hoch sein. Die Abwesenheit dieser Fragmente ist wünschenswert, wenn die Plasmid DNA sequenziert oder mit PCR amplifiziert werden soll. Andere Methoden um kleine Nukleinsäurefragmente zu entfernen sind die Sephacryl S-1000 Chromatographie und Fällung mit Lithiumchlorid (LiCl). LiCl ist ein stark dehydrierendes Mittel, das die Löslichkeit von RNA reduziert und Proteine von Chromatin entfernt (Kondo et al. 1979), deshalb können Kontaminationen von hoch-molekularer RNA und Proteinen aus der Plasmid-Präparation durch Fällung entfernt werden.

Der Nachweis von Mollicutes neben den Schritten der Probengewinnung und DNA Extraktion umfasst auch die Schritte der Amplifikation mittels PCR, Hybridisierung, Waschen, Scannen sowie Evaluierung des Ergebnis gemäß CytoInspect® der Firma Greiner Bio-One GmbH.

Es können auch virale und prokaryontische Nukleinsäuren von pflanzlicher und fungaler DNA getrennt werden, wobei man vor oder zusätzlich zur alkalischen Lyse noch andere Lyseschritte, wie beispielsweise eine mechanische Lyse, braucht. Die biologische Probe, insbesondere Zellsuspension, wird mit dem Resuspensionspuffer resuspendiert und zum Lysat wird der Neutralisationspuffer zugegeben und somit die pflanzliche und fungale DNA entsprechend dem erfindungsgemäßen Verfahren getrennt.

Auf welchen theoretischen Grundlagen die erfindungsgemäße Trennung der viralen und prokaryontischen Nukleinsäure, insbesondere DNA, von der eurkaryontischen DNA basiert ist noch nicht restlos geklärt. Mögliche Erklärungsvarianten sind wie bereits vorab schon erwähnt die Größe, die Linearität und Proteinkoppelung der eukaryontischen DNA.

Neben der vorab beschriebenen Trennung der viralen und prokaryontischen Nukleinsäure von eukaryontischer Nukleinsäure ist auch eine Detektion der viralen und prokaryontischen Nukleinsäure in Zellkulturüberständen, Zellkulturmedien, proteinreichen Proben mit hohen BSA-Konzentrationen und Allantoisflüssigkeit (Hühnerei) möglich.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele zeigen mögliche Ausführungsvarianten des erfindungsgemäßen Verfahrens bzw. der Verwendung des Kits, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Die den eigenständigen erfinderischen Lösungen zugrundeliegende Aufgabe kann der Beschreibung entnommen werden.

## Patentansprüche

1. Verfahren zur Trennung viraler und/oder prokaryontischer Nukleinsäuren von eukaryontischen Nukleinsäuren aus einer biologischen Probe, insbesondere eukaryontischen Zellsuspension, umfassend die Schritte in der Reihenfolge a) Resuspendieren der Zellen einer Zellsuspension bzw. der Zellen nach Zentrifugation der Zellen in Gegenwart eines ChelatBildners, insbesondere EDTA oder EDTA in Kombination eines Saccharids, b) Lyse der Zellen durch chemische Lyse, wie alkalische Lyse, enzymatische Lyse und/oder Kochlyse und/oder mechanische Lyse, wobei die Lyse in Gegenwart eines Detergens, insbesondere SDS, und einer Base durchgeführt wird, c) Neutralisation des Zelllysats in Gegenwart zumindest eines Salzes bzw. Stoffes, das/der die Löslichkeit von Proteinen reduziert, insbesondere eines kosmotropen Salzes, insbesondere Kalium- oder Natriumacetat bzw. Ammoniumsulfat, wo die Proteinfällung erfolgt, und d) Abtrennen der ausgefällten eukaryontischen Nukleinsäuren sowie Gewinnung der viralen und/oder prokaryontischen Nukleinsäuren, die in Lösung bleiben.

2. Verfahren nach Anspruch 1, **dadurch gekennzeichnet, dass** die viralen und prokaryontischen Nukleinsäuren nach der Neutralisation gereinigt, insbesondere präzipitiert und/oder zentrifugiert, werden.

3. Verfahren nach Anspruch 1 oder 2, **dadurch gekennzeichnet, dass** durch eine Amplifikation der prokaryontischen Nukleinsäuren aus einer Zellsuspension mit einer Zelldichte von 10^5 bis 10^10 Zellen/ml eine Nachweisgrenze <10 cfu/ml bzw. <10 Kopien/ml erreicht wird.

4. Verfahren nach einem der Ansprüche 1 bis 3, **dadurch gekennzeichnet, dass** Mollicutes, insbesondere Mycoplasma sp., Acholeplasma sp., Spiroplasma sp. und Ureaplasma sp., DNA von eukaryontischer DNA durch alkalische Lyse separiert wird.

5. Verfahren nach Anspruch 4, **dadurch gekennzeichnet, dass** Mollicutes, insbesondere Mycoplasma sp., Acholeplasma sp., Spiroplasma sp. und Ureaplasma sp., DNA aus einer Zellsuspension mit einer Zelldichte von bis 10^10 Zellen/ml extrahiert und nachgewiesen wird.

6. Verfahren nach Anspruch 4 oder 5, **dadurch gekennzeichnet, dass** eine Nachweisgrenze von 5 colony forming units für Mollicutes in 10 ml Zellsuspension mit einer Zellzahl von 10^8 Zellen erreicht wird.

7. Verwendung eines Kits umfassend einen Resuspensionspuffer mit Chelatbildner und gegebenenfalls ein Saccharid und RNAse, Lysepuffer umfassend zumindest eine Base und ein Detergens und Neutralisationspuffer zur Trennung viraler und prokaryontischer Nukleinsäuren von eukaryontischen Nukleinsäuren aus einer biologischen Probe, insbesondere eukaryontischen Zellsuspension.

8. Verwendung des Kits nach Anspruch 7, **dadurch gekennzeichnet, dass** der Neutralisationspuffer zumindest ein Salz bzw. Stoff enthält, das/der die Löslichkeit von Proteinen reduziert, insbesondere ein kosmotropes Salz ausgewählt aus einer Gruppe umfassend Kalium- oder Natriumacetat, Kalium- oder Natriumphosphat, MgSO₄, NaCl, Na₂SO₄, (NH₄)₂SO₄, enthält.

9. Verwendung des Kits nach einem der Ansprüche 7 oder 8 zur Isolierung von Mollicutes DNA aus einer eukaryontischen Zellsuspension mittels alkalischer Lyse.

## Claims

1. A method for separating viral and/or prokaryotic nucleic acids from eukaryotic nucleic acids from a biological sample, in particular eukaryotic cell suspension, comprising the following steps in the order of a) resuspending the cells of a cell suspension or the cells after centrifugation of the cells in the presence of a chelating agent, in particular EDTA or EDTA in combination with a saccharide, b) lysing the cells by chemical lysis, such as alkaline lysis, enzymatic lysis and/or boiling lysis and/or mechanical lysis, wherein the lysis is performed in the presence of a detergent, in particular SDS, and a base, c) neutralising the cell lysate in the presence of at least one salt or substance that reduces the solubility of proteins, in particular of a kosmotropic salt, in particular potassium acetate or sodium acetate or ammonium sulphate, where the protein precipitates, and d) separating the precipitated eukaryotic nucleic acids and recovering the viral and/or prokaryotic nucleic acids remaining in solution.

2. The method according to claim 1, **characterised in that** the viral and prokaryotic nucleic acids are purified following the neutralisation, in particular are precipitated and/or centrifuged.

3. The method according to claim 1 or 2, **characterised in that**, by an amplification of the prokaryotic nucleic acids from a cell suspension with a cell density of 10^5 to 10^10 cells/ml, a detection limit <10 cfu/ml or <10 copies/ml is achieved.

4. The method according to one of claims 1 to 3, **characterised in that** mollicutes, in particular mycoplasma sp., acholeplasma sp., spiroplasma sp. and ureaplasma sp., DNA is separated from eukaryotic DNA by alkaline lysis.

5. The method according to claim 4, **characterised in that** mollicutes, in particular mycoplasma sp., acholeplasma sp., spiroplasma sp., and ureaplasma sp., DNA is extracted from a cell suspension having a cell density of up to 10^10 cells/ml and is detected.

6. The method according to claim 4 or 5, **characterised in that** a detection limit of 5 colony forming units for mollicutes in 10 ml cell suspension with a cell count of 10^8 cells is achieved.

7. Use of a kit comprising a resuspension buffer with chelating agent and optionally a saccharide and RNAse, lysis buffer comprising at least one base and a detergent and neutralisation buffer for separation of viral and prokaryotic nucleic acids from eukaryotic nucleic acids from a biological sample, in particular eukaryotic cell suspension.

8. Use of the kit according to claim 7, **characterised in that** the neutralisation buffer contains at least one salt or substance that reduces the solubility of proteins, in particular a kosmotropic salt selected from a group comprising potassium acetate or sodium acetate, potassium phosphate or sodium phosphate, MgSO₄, NaCl, Na₂SO₄, (NH₄)₂SO₄.

9. Use of the kit according to one of claims 7 or 8 for isolating mollicutes DNA from a eukaryotic cell suspension by means of alkaline lysis.

## Revendications

1. Procédé pour séparer des acides nucléiques viraux et/ou procaryotes d'acides nucléiques eucaryotes d'un échantillon biologique, notamment d'une suspension eucaryote de cellules, comprenant, dans l'ordre, les étapes a) remettre en suspension les cellules d'une suspension de cellules ou des cellules après centrifugation des cellules en présence d'un chélateur, notamment EDTA ou EDTA en combinaison avec un saccharide, b) lyse des cellules par lyse chimique telle que lyse alcaline, lyse enzymatique et/ou lyse par ébullition et/ou lyse mécanique, la lyse étant effectuée en présence d'un détergeant, notamment SDS, et d'une base, c) neutralisation du lysat de cellules en présence d'au moins un sel ou d'un agent réduisant la solubilité de protéines, notamment d'un sel cosmotropique, notamment acétate de potassium ou de sodium ou sulfate d'ammonium, où la précipitation des protéines se produit, et d) séparation des acides nucléiques eucaryotes précipités et obtention des acides nucléiques viraux et/ou procaryotes qui restent en suspension.

2. Procédé selon la revendication 1, **caractérisé en ce que** les acides nucléiques viraux et/ou procaryotes sont nettoyés, notamment précipités et/ou centrifugés, après la neutralisation.

3. Procédé selon la revendication 1 ou 2, **caractérisé en ce que**, par une amplification des acides nucléiques procaryotes, on obtient d'une suspension de cellules ayant une densité de cellules de 10^5 à 10^10 cellules/ml, une limite de détection <10 cfu/ml ou <10 copies/ml.

4. Procédé selon l'une des revendications 1 à 3, **caractérisé en ce que** des ADN de type mollicutes, notamment mycoplasme sp., acholeplasme sp., spiroplasme sp. et uréaplasme sp. sont séparés d'ADN eucaryote par lyse alcaline.

5. Procédé selon la revendication 4, **caractérisé en ce que** des ADN de type mollicutes, notamment mycoplasme sp., acholeplasme sp., spiroplasme sp. et uréaplasme sp. sont extraits et détectés d'une suspension de cellules ayant une densité de cellules de jusqu'à 10^10 cellules/ml.

6. Procédé selon la revendication 4, **caractérisé en ce qu'**une limite de détection de 5 cfu est atteinte pour des mollicutes dans 10 ml d'une suspension de cellules ayant un nombre de cellules de 10^8 cellules.

7. Utilisation d'un kit comprenant un tampon de remise en suspension avec chélateur et, le cas échéant, un saccharide et une RNase, tampon de lyse comprenant au moins une base et un détergent et un tampon de neutralisation pour la séparation d'acides nucléiques viraux et/ou procaryotes d'acides nucléiques eucaryotes d'un échantillon biologique, notamment d'une suspension eucaryote de cellules.

8. Utilisation d'un kit selon la revendication 7, **caractérisée en ce que** le tampon de neutralisation comprend au moins un sel ou un agent qui réduit la solubilité de protéines, notamment un sel cosmotrope choisi dans un groupe comprenant acétate de potassium ou de sodium, phosphate de potassium ou de sodium, MgSO₄, NaCl, Na₂SO₄, (NH₄)₂SO₄.

9. Utilisation d'un kit selon la revendication 7 ou 8 pour l'isolation d'ADN de type mollicute d'une suspension eucaryote de cellules à l'aide d'une lyse alcaline.
